# EUROPÄISCHE PATENTSCHRIFT

(11) **EP 1 711 144 B1**
(45) Veröffentlichungstag und Bekanntmachung des Hinweises auf die Patenterteilung: **03.08.2011**
(21) Anmeldenummer: 05700346.9
(22) Anmeldetag: 01.02.2005
(51) Int. Cl.: A61F 7/10

(54) **Personenkühlelement, insbesondere für Patienten**
Personal cooling element, in particular for patients
Élément de refroidissement d'une personne, notamment pour des patients

(30) Priorität: 05.02.2004 CH 1722004
(43) Veröffentlichungstag der Anmeldung: 18.10.2006
(73) Patentinhaber: Unico Swiss Tex GmbH, 6053 Alpnachstad (CH)
(72) Erfinder: SELM, Bärbel, CH-9305 Berg/SG (CH); WÜST, Benno, CH-9425 Thal (CH); WEDER, Markus, CH-9428 Walzenhausen (CH)
(74) Vertreter: Felder, Peter
(86) Internationale Anmeldenummer: PCT/CH2005/000051
(87) Internationale Veröffentlichungsnummer: WO 2005/074846

(56) Entgegenhaltungen:
- EP-A- 0 885 601
- DE-A- 3 902 233
- GB-A- 2 248 675
- US-A- 5 433 083

## Beschreibung

### Technisches Gebiet

Die Erfindung betrifft ein Personenkühlelement, insbesondere für Patienten, nach dem Oberbegriff des Anspruchs 1

### Stand der Technik

Personenkühlelemente, insbesondere kühlende Kleidungsstücke, sind in einer Vielzahl bekannt und für unterschiedlichste Einsatzgebiete konzipiert worden. Sowohl für Sportler als auch für Arbeiter, die heissen Wärmequellen ausgesetzt sind, sowie für Patienten zur Linderung von Krankheitssymptomen und/oder zu Therapiezwecken ist eine gute und effiziente Kühlung von bestimmten Körperpartien notwendig.

In der US 5,269,369 ist ein Kleidungsstück beschrieben, das einen thermischen Ausgleich zwischen kühleren und heisseren thermischen Körperpartien der tragenden Person schafft. Dabei wird über Wärmerohre Wärme von den heisseren, kühlungsbedürftigen Körperpartien zu kälteren, wärmungsbedürftigen Körperpartien geleitet. Die Wärmerohre sind elastisch ausgebildet und an dem Kleidungsstück angebracht. Die Wärmerohre der US 5,269,369 können aber auch mit einem externen Kühl- oder Wärmelement in Verbindung sein, um eine gezielte Kühlung oder Erwärmung bestimmter Körperpartien zu erreichen. Es wird in der US 5,269,369 vorgeschlagen, ein solches Kleidungsstück nicht nur bei thermisch extremen Umweltbedingungen einzusetzen, wie dies beispielsweise beim Tauchen in grosser Tiefe oder in Polarregionen der Fall ist, sondern auch bei Menschen, die unter bestimmten Krankheiten leiden, insbesondere Multiple Sklerose (nachfolgend "MS").

Um eine ausreichende Kühlwirkung über längere Zeit mit dem in der US 5,269,369 beschriebenen Kleidungsstück zu erzielen, genügt es nicht, die Wärme von heisseren Körperpartien zu kälteren Körperpartien über die Wärmerohre zu leiten. Vielmehr ist ein dauernder Wärmeentzug für einen Grossteil des Bewegungsapparats notwendig, so dass die Wärmerohre der US 5,269,369 mit einer mitzuführenden Wärmesenke in Verbindung stehen müssen. Ein solches Kleidungsstück ist sehr schwer und daher nur für kräftige Menschen geeignet. Eine Anwendung für Kinder oder ältere Menschen ist daher nicht möglich. Überdies ist das ständige Mitführen der Wärmesenke für die das Kleidungsstück tragende Person mühsam und schränkt diese in ihrer Bewegungsfreiheit ein.

Die EP 1,273,277 A2 beschreibt ein Bekleidungsstück zur Therapie von MS-Patienten, wobei das Bekleidungsstück als Schläuche ausgestaltete Kühlelemente aufweist, welche an der Innenseite des Bekleidungsstücks eine Kühlwirkung entfalten. Ein solches Kleidungsstück muss über Anschlüsse mit einem Kühlaggregat verbunden sein, um Kühlmedium zu den Kühlelementen zu führen, respektive von diesen abzuführen. Auch dieses Kleidungsstück ist wie jenes der US 5,269,369 schwer und aufgrund seiner Sperrigkeit bzw. Steifheit unbequem. Für Kinder, ältere Menschen und insbesondere auch für Patienten erscheint das kühlende Bekleidungsstück wenig geeignet. Eine Ausgestaltung als Ganzkörperanzug, die für gewisse Anwendungen erwünscht wäre, ist aufgrund des hohen Gewichts und des schlechten Tragkomforts kaum gangbar. Überdies erfüllt ein solches Kleidungsstück die Anforderungen bezüglich Ästhetik aufgrund seiner steifen Struktur und seiner Unförmigkeit nicht.

Weitere gängige Kühlmethoden für MS-Patienten wie auch für andere Personen reichen von nassen Umschlägen bis zu mitgeführten Kleinventilatoren. Nasse Umschläge sind zwar recht wirksam, führen aber zu unerwünschter Nässung der Person und der übrigen Bekleidung und sind deshalb nur in einigen wenigen Situationen brauchbar. Kleinventilatoren und dergleichen sind wenig effektiv, bedingen eine gewisse Einschränkung der Bewegungsfreiheit und sind zudem aufgrund der Geräuschentwicklung unangenehm. Besonders für Patienten mit temperatursensitiven Leiden des Bewegungsapparats sind somit die bekannten Kühlvorrichtungen und -methoden für eine aktive Teilnahme am sozialen wie auch beruflichen Leben nicht zufrieden stellend.

Ein weiteres Personenkühlelement ist in der DE 39 02 233 A1 beschrieben. Dieses ist als beutelförmige Kühlvorrichtung zur Füllung mit einer Kühlflüssigkeit ausgebildet und besteht auf seiner der Kühlung dienenden Seite aus einem mit der Kühlflüssigkeit in Berührung stehenden, flüssigkeitsdichten, dampfdurchlässigen Material. Letzteres ist auf der nicht mit der Kühlflüssigkeit in Berührung stehenden Seite von einer hydrophilen, wasserabsorbierenden Materialschicht und darüber von einer hydrophoben, wasserleitenden Materialschicht überlagert, wobei all diese Materialien bielastische Werkstoffe sind. Die hydrophobe, wasserleitende Materialschicht ist die dem zu kühlenden Körperteil am nächsten liegende Schicht und dient dazu, Wasser von der Körperhaut in die hydrophile wasserabsorbierende Materialschicht zu transportieren. Der Kühleffekt ergibt sich primär durch den Vorrat an Kühlflüssigkeit, welche beim Einfüllen typischerweise eine Temperatur von -5 bis 7°C aufweist und damit ein Kältereservoir bildet. Eine zusätzliche Kühlwirkung ergibt sich zudem durch Verdampfung des Kühlmittels. Dabei tritt Kühlmitteldampf aus dem Beutel durch das flüssigkeitsdichte, dampfdurchlässige Material hindurch und gelangt in die daran angrenzende hydrophile wasserabsorbierende Materialschicht und kann letztlich in die Umgebung entweichen. Das bekannte Personenkühlelement ist somit in der Art einer herkömmlichen Bettflasche aufgebaut, unterscheidet sich davon aber dadurch, dass Kühlmitteldampf aus dem Beutel treten kann.

### Darstellung der Erfindung

Zweck der Erfindung ist es, ein Personenkühlelement der eingangs genannten Art zu verbessern und ein Verfahren zur Kühlung von Körperpartien anzugeben.

Die gestellten Aufgaben werden durch das im Anspruch 1 definierte Personenkühlelement gelöst.

Das erfindungsgemässe Personenkühlelement weist mindestens eine Kühlzone auf, wobei elastische Mittel vorhanden sind, um die Kühlzone gegen die Körperoberfläche einer das Personenkühlelement tragenden Person vorzuspannen. Dadurch, dass die Kühlzone dreilagig ausgebildet ist mit einer körperzugewandten Innenschicht, einer körperabgewandten Aussenschicht sowie einem dazwischen angeordneten Verdunstungsbereich, wobei die Innenschicht und die Aussenschicht je aus einem wasserdichten und wasserdampfdurchlässigen Material gebildet sind, wobei Wasserzuführmittel vorhanden sind, um den Verdunstungsbereich mit flüssigem Wasser zu versorgen, wobei der Verdunstungsbereich aus einem hydrophilen Material gebildet und ist und die Innenschicht für Hantkontakt vorgesehen ist ergibt sich ein kompaktes, angenehm zu tragendes, einfach aufgebautes und leistungsfähiges Personenkühlelement.

Demnach können zu kühlende Körperpartien einer das Personenkühlelement tragenden Person unter Ausnutzung der Verdunstungsenergie des in den Verdunstungsbereich zugeführten Wassers gekühlt werden. Im Unterschied zur DE 39 02 233 A1 ist beim erfindungsgemässen Personenkühlelement die für den Hautkontakt vorgesehene Seite - hier als Innenschicht bezeichnet - für flüssiges Wasser undurchlässig. Dadurch zeichnet sich das erfindungsgemässe Personenkühlelement im Vergleich zu demjenigen aus der DE 39 02 233 A1 durch einen wesentlich höheren Tragekomfort aus, da die wasserdichte Innenschicht ein Benetzen der Haut der das Personenkühlelement tragenden Person verhindert. Ein derartiges Benetzen wäre nicht nur unangenehm, sondern würde überdies aufgrund der nicht unerheblichen Wasseraufnahme durch die Haut zu einem Verlust von Kühlwasser führen. Gleichzeitig kann aber die von der Haut abgegebene Feuchtigkeit nach aussen weggeführt werden, d.h. der körpereigene Kühlmechanismus des Schwitzens ist trotz des darüber liegenden Kühlelements weiterhin möglich. Durch die dreilagige Ausbildung mit zwischen Innenschicht und Aussenschicht befindlichem Verdunstungsbereich, der durch die Wasserzuführmittel mit Wasser versorgbar ist, kann die den Verdunstungsbereich bildende Zwischenschicht sehr dünn sein. Der Verdunstungsbereich soll lediglich sicherstellen, dass bei Kühlungsbedarf ständig eine wenn auch nur geringe Menge flüssiges Wasser vorhanden ist, das durch Verdunsten die erforderliche Kühlung bewirkt. Dadurch, dass die auf der körperabgewandten Seite des Verdunstungsbereichs befindliche Aussenschicht im wesentlichen freiliegend ist und insbesondere nicht durch ein beutelartiges Reservoir wie in der DE 39 02 233 A1 überlagert ist, kann Wasserdampf leicht durch die Aussenschicht austreten. Dies erlaubt eine vergleichsweise hohe Verdunstungsrate und damit trotz einfachstem Aufbau des Kühlelementes eine hervorragende Kühlleistung.

Das erfindungsgemässe Personenkühlelement erlaubt eine effiziente Kühlung von Körperpartien und kann so die Befindlichkeit der das Personenkühlelement tragenden Person erheblich steigern. Insbesondere MS-Patienten können dank der effizienten und mit einfachen Mitteln ausführbaren Kühlung ohne wesentliche Einbusse an Komfort ohne Erschöpfungspause grössere Distanzen zurücklegen als ohne Personenkühlelement. Damit lässt sich die Lebensqualität für Menschen mit bestimmten Leiden deutlich erhöhen. Andererseits kann das Personenkühlelement aber auch bei der Ausübung gewisser sportlicher oder beruflicher Aktivitäten verwendet werden. Insbesondere kann damit die körpereigene Kühlung des Schwitzens ersetzt oder aber ergänzt werden. Nebst Wasser als bevorzugtem Kühlmittel ist es auch denkbar, andere wasserbasierte Kühlmittel wie beispielsweise ein Wasser-AlkoholGemisch zu verwenden. Im vorliegenden Zusammenhang sind dann die Begriffe "wasserdicht", für "Wasser undurchlässig" und "wasserdampfdurchlässig" sinngemäss zu verstehen als dicht bzw. undurchlässig für das flüssige Gemisch bzw. als durchlässig für Dämpfe des besagten Gemisches.

Mit dem erfindungsgemässen Personenkühlelement ist eine effiziente Kühlung von ausgewählten Körperpartien, insbesondere von Extremitäten und Muskelpartien möglich. Es ist davon auszugehen, dass die damit einhergehende geringfügige Senkung der Bluttemperatur zu einer Temperaturreduktion im Zentralnervensystem führt, die bei MS-Patienten eine Verbesserung der Leistungsfähigkeit ergibt.

Vorteilhafterweise weisen Innenschicht und Aussenschicht eine hohe Wärmeleitfähigkeit auf. Um ein enges Hautanliegen der Kühlzone zu garantieren, kann die Kühlzone beispielsweise mit elastischen Bändern ausgestattet sein. Es ist auch möglich, die Innenschicht und/oder die Aussenschicht selbst aus einem elastisch wirkenden Material zu bilden. Ein enges Hautanliegen der Kühlzone ist für eine optimale Kühlung imperativ.

Die Innenschicht kann als gut hydrophobiertes Gewebe oder als Membrane aus Polyetherester (beispielsweise Sympatex™) oder aus PTFE (beispielsweise Gore-Tex™) gebildet sein. Die Aussenschicht ist vorteilhafterweise aus einer dünneren Membrane aus Polyetherester (beispielsweise Sympatex™) gebildet.

Das erfindungsgemässe Verfahren zum Kühlen von Körperpartien besteht darin, dass man das Personenkühlelement auf eine zu kühlende Körperpartie hautnah anbringt und gegen diese vorspannt und dann den Verdunstungsbereich der Kühlzone kontinuierlich oder intermittierend, d.h. nach Bedarf mit Wasser versorgt. Dabei wird unter Ausnutzung der Verdunstungsenergie des Wassers eine Abkühlung der mit der Kühlzone in thermischem Kontakt stehenden Körperpartie auf einfache Weise erreicht. Die Kühlwirkung tritt schnell ein, und deren Dauer lässt sich durch die zugeführte Wassermenge steuern: sobald alles Kühlwasser verdunstet ist, hört die Kühlwirkung auf. Somit kann in Phasen, in denen keine Kühlung erforderlich ist, das trockene Personenenkühlelement mit gutem Komfort weiterhin getragen werden.

Vorteilhafte Ausgestaltungen der Erfindung sind in den Ansprüchen 2 bis 8 definiert. der Verdunstungsbereich aus einem Erfindungsgemäß ist hydrophilen Material gebildet. Dadurch ist eine gute Aufnahme und Verteilung von Wasser in dieser Schicht möglich.

Um die Aufnahme und Verteilung des Wassers in dem Verdunstungsbereich weiter zu verbessern, ist eine Ausgestaltung vorteilhaft, bei welcher der Verdunstungsbereich ein Kanalsystem aufweist. Letzteres erstreckt sich zweckmässigerweise auf die ganze Fläche des Verdunstungsbereichs und ist vorteilhafterweise direkt mit einer Eintrittsöffnung für zugeführtes Kühlwasser verbunden. Dadurch kann das Wasser direkt in das Kanalsystem gelangen, womit eine optimale Verteilung des Wassers in dem Verdunstungsbereich erzielt wird.

Vorteilhafterweise weist die Aussenschicht eine Dicke von 1 bis 5 µm auf. Als vorteilhaft hat es sich zudem erwiesen, wenn die Innenschicht eine Dicke von 10 bis 20 µm aufweist.

Um ein Abfliessen des dem Verdunstungsbereich zugeführten Wassers zu verhindern, sind die Aussenschicht und die Innenschicht zu einem seitlichen Abschluss des Verdunstungsbereichs verbunden. Dadurch ist der ganze Verdunstungsbereich mittels der Innenschicht und der Aussenschicht nach Aussen abgeschlossen. Zugeführtes Wasser kann im Wesentlichen lediglich als Wasserdampf über die Aussenschicht abgegeben werden.

Das Personenkühlelement kann beispielsweise als elastische Binde oder aber als Bekleidungsstück ausgestaltet sein. Insbesondere kann es sehr dünn, beispielsweise in der Art eines Strumpfs ausgebildet sein und wird in der Regel unter normaler Kleidung getragen, welche jedoch eine möglichst gute Abführung des verdunsteten Wassers gewährleisten sollte. Für eine an einer Nerven- oder Muskelkrankheit leidende Person ist es vorteilhaft, wenn das Personenkühlelement als dünner elastischer Anzug ausgestaltet ist. Insbesondere kann dieser Anzug als Ganzkörperanzug oder aber als Hose und/oder Oberteil ausgebildet sein. Dadurch ist es möglich, eine Kühlzone für grosse Teile der Körperoberfläche vorzusehen und somit eine gute Abkühlung der massgeblichen Körperregionen zu erreichen.

Im Weiteren kann es vorteilhaft sein, die Kühlzone lediglich zum Anliegen an einer oder mehreren ausgewählten Körperpartien auszubilden. Dadurch ist eine gezielte Kühlung wärmeempfindlicher Körperpartien, insbesondere bei lokalen Verbrennungen oder Ischiasnervleiden, möglich.

Besonders vorteilhaft ist eine Ausgestaltung, bei der die Wasserzuführmittel ein mit einer Pumpe verbundenes Wasserleitungssystem umfassen. Dadurch kann eine gleichmässige Verteilung der Kühlfüssigkeit entlang des Verdunstungsbereichs der Kühlzone mit minimalem technischem Aufwand erreicht werden. Sobald die das Personenkühlelement tragende Person starker Hitzeeinstrahlung ausgesetzt ist und/oder die Notwendigkeit einer verbesserten Kühlung ihres Körpers wahrnimmt, kann sie entweder automatisch mittels einer elektrischen Pumpe oder von Hand mittels einer Dispenserpumpe Kühlflüssigkeit der Kühlzone zuführen.

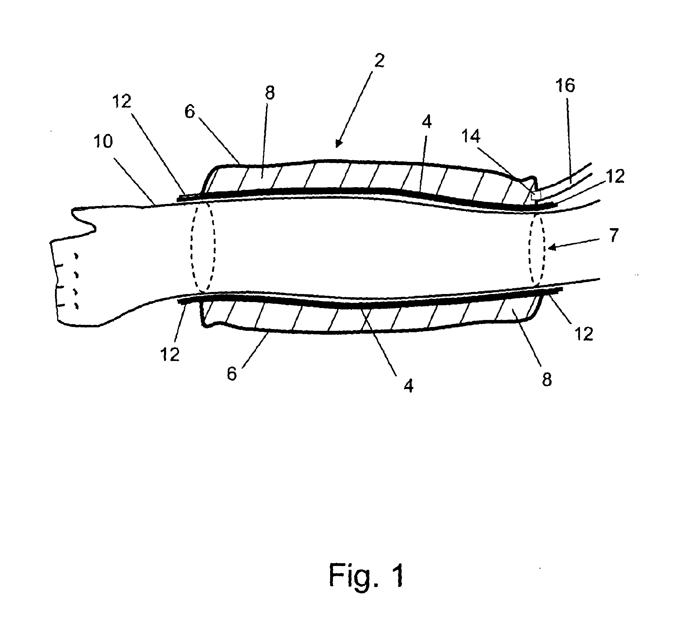

### Kurze Beschreibung der Zeichnung

Ein Ausführungsbeispiel der Erfindung wird nachfolgend anhand der einzigen Figur näher beschrieben, welche einen Ausschnitt einer Kühlzone eines hautnah am Unterarm getragenen Personenkühlelements im Längsschnitt zeigt.

### Wege zur Ausführung der Erfindung

Die in der Figur ausschnittsweise gezeigte dreilagige Kühlzone 2 ist aus einer wasserdichten aber wasserdampfdurchlässigen Innenschicht 4, einer wasserdichten aber wasserdampfdurchlässigen Aussenschicht 6 sowie einem zwischen diesen Schichten befindlichen hydrophilen Verdunstungsbereich 8 gebildet. Letzterer ist zur Befüllung mit Wasser oder einem ähnlichen Kühlmittel vorgesehen und ist vorzugsweise aus einer hydrophilen Textillage gebildet, welche durch die Innenschicht 4 gegenüber der Körperoberfläche 10 einer das Personenkühlelement tragenden Person abgegrenzt ist. Vorzugsweise ist die Innenschicht 4 wie auch die Aussenschicht 6 aus je einer elastischen Membrane gebildet. Die elastischen Schichten 4, 6 wirken auf einfache Weise als elastische Vorspannmittel 7, um ein enges Hautanliegen der Kühlzone 2 an der Haut zu gewährleisten. Alternativ können jedoch eigenständige, d.h. von der Innen- und Aussenschicht unabhängige Vorspannmittel, beispielsweise elastische Bänder, Schnüre oder dergleichen verwendet werden.

Die Innenschicht 4 hat eine Dicke von 10 bis 20 µm, um einerseits eine gute Wärmeleitung von der Haut zu dem Verdunstungsbereich zu gewährleisten und andererseits ein Durchdringen von flüssigem Wasser zu vermeiden. Gleichzeitig ist die Innenschicht für Wasserdampf durchlässig, wodurch der Tragkomfort des Kühlelements verbessert und die körpereigene Kühlwirkung des Schwitzens ermöglicht wird. Bei einer Sympatex-Membrane mit einer Schichtdicke von 15 µm beträgt der nach ISO 11092 gemessene Wasserdampf-Durchgangswiderstand (Ret-Wert) im trockenen Zustand ungefähr 7 m²Pa/W.

Die Aussenschicht 6 weist eine Dicke von ungefähr 1 bis 5 Mikrometern auf und erstreckt sich auf seitliche Abschlüsse 12 des Verdunstungsbereichs, um ein Ausfliessen von im Verdunstungsbereich 8 befindlichem Wasser zu vermeiden. Die Aussenschicht 6 ist wasserdicht aber für Wasserdampf hoch durchlässig und garantiert somit ein gutes Abführen des in dem Verdunstungsbereich 8 verdunsteten Wassers. Bei einer Sympatex-Membrane mit einer Schichtdicke von 5 µm beträgt der Ret-Wert im trockenen Zustand ungefähr 2 m²Pa/W.

Bei einem Ausführungsbeispiel des Personenkühlelementes ist die körperzugewandte Innenschicht 4 aus einer Sympatex-Membrane mit einer Schichtdicke von 15 µm, die körperabgewandte Aussenschicht 6 durch eine Sympatex-Membrane mit einer Schichtdicke von 5 µm und der zwischen diesen Schichten liegende Verdunstungsbereich 8 durch eine hydrophile Polyesterlage mit einer Schichtdicke von ca. 0.5 mm gebildet. Für das dreilagige Gesamtgebilde wurde im trockenen Zustand ein Ret-Wert von ungefähr 10 m²Pa/W gemessen. Da jedoch bei der praktischen Anwendung die Verdunstungsrate des Kühlmittels und damit auch die Kühlleistung vornehmlich durch die Passage von Dampf aus der hydrophilen Mittelschicht durch die Aussenschicht 6 begrenzt wird, kommt primär der Ret-Wert der Aussenschicht zum Tragen, welcher mit ungefähr 2 m²Pa/W in vorteilhafter Weise sehr niedrig ist.

Wasser kann beispielsweise mittels eines flexiblen Röhrchens 14 und einer nicht dargestellten Pumpe durch eine Öffnung 16 dem Verdunstungsbereich 8 zugeführt werden. Vorteilhafterweise wird hierfür eine kleine handbetätigte Dispenserpumpe verwendet, mit welcher nach Bedarf Portionen von einigen Millilitern Wasser zugeführt werden können. Aufgrund der Hydrophilie des Verdunstungsbereichs 6 verteilt sich das Wasser gleichmässig darin und ermöglicht dadurch eine gleichmässige Kühlung der durch die Kühlzone 2 abgedeckten Körperpartie. Durch das Verdunsten des Wassers und den Austritt von Wasserdampf durch die Aussenschicht 6 wird dem Verdunstungsbereich 8 Wärme entzogen, was aufgrund der körpernahen Anordnung des Verdunstungsbereichs 8 und der guten Wärmeleitung der Innenschicht 4 zur Kühlung der entsprechenden Körperpartie führt. Vorteilhafterweise besteht der Verdunstungsbereich aus einem netzartigen Gewebe oder einem anderen Gebilde mit grosser effektiver Oberfläche, um eine möglichst effiziente Verdunstung zu bewirken.

Das in der Figur ausschnittsweise gezeigte Kühlelement kann sowohl zur Kühlung von kleineren Körperpartien, beispielsweise für Patienten mit lokalen Verbrennungen, oder für den ganzen Körper verwendet werden. Für eine Ganzkörperkühlung werden sowohl Hose als auch Oberteil vollständig aus der in Figur im Ausschnitt gezeigten Kühlzone gebildet. Sowohl die Hose als auch das Oberteil bilden dann je mindestens eine Kühlzone.

Für den Verdunstungsbereich kommen verschiedene hydrophile Fasermaterialien in Frage, wobei einige Polymerfasern vor der Verwendung hydrophiliert werden müssen. Bekannte Verfahren hierfür umfassen beispielsweise eine Plasmabehandlung. Vorteilhaft ist es, wenn diese Mittelschicht des Kühlelementes aus einem saugfähigen Material besteht, welches bereits aufgrund des Garns und der Konstruktion eine gute Hydrophilie aufweist.

Bei der Erprobung der Kühlfähigkeit einer kühlenden Hose ergab eine Zugabe von 15 g Wasser eine Abkühlung der Oberschenkeltemperatur um ca. 7°C während einer Zeitdauer von einer Stunde. Die besten Kühleffekte ergaben Polyestermaterialien, die sehr hydrophil waren. Diese nehmen die Feuchtigkeit in der Fläche rasch auf und können diese noch in Hautnähe zum Verdunsten bringen. Denn bereits ein zusätzlicher Abstand von 1 mm zwischen dem Verdunstungsort und der Körperoberfläche führt zu einer erheblichen Einbusse der Kühlwirkung und ist demnach sehr unerwünscht.

Da zur effektiven Nutzung des Verdunstungseffektes eine möglichst gute Wärmeleitfähigkeit zwischen körperabgewandter Verdunstungszone und Körperoberfläche benötigt wird, sollte das Textilmaterial selbst, d.h. bereits im trockenen Zustand, eine möglichst gute Wärmeleitfähigkeit resp. geringe Wärmeisolation aufweisen. Die hier verwendeten Sympatex-Membranen weisen im trockenen Zustand eine Wärmeleitfähigkeit von 0.048 W/mK auf, welche im nassen Zustand sogar auf 0.244 W/mK, also auf das Fünffache ansteigt. Das dreilagige Gesamtgebilde in obigem Ausführungsbeispiel besitzt im trockenen Zustand einen Wärmedurchgangswiderstand mit einem Rct-Wert nach ISO 11092 von ungefähr 5.8x10⁻³ m²K/W auf. Dieser Wert ist deutlich kleiner als jener von gängiger Unterwäsche, welche typischerweise Rct-Werte von 25 bis 30x10⁻³ m²K/W aufweist. Eine noch bessere Wärmeleitfähigkeit könnte gewünschtenfalls durch Ionendotierung der Fasern erreicht werden.

Für manche Anwendungen, beispielsweise bei MS-Patienten, ist es in der Praxis erwünscht, zumindest über Teilen des Kühlelementes ein Kleidungsstück zu tragen. Dabei ist es empfehlenswert, eine nicht anliegende, d.h. locker fallende Aussenbekleidung zu verwenden. Dadurch wird erreicht, dass der aus dem Kühlelement austretende Wasserdampf möglichst gut abgeführt wird und damit weiterer Wasserdampf nachfolgen kann. Zweckmässigerweise sollte hierfür die Aussenschicht 6 eine möglichst hohe Durchlässigkeit für Wasserdampf aufweisen. Da die Aussenschicht 6 für flüssiges Wasser jedoch undurchlässig ist, wird nicht nur ein Verlust von flüssigem Kühlmittel, sondern auch ein unerwünschtes Nässen der Aussenbekleidung vermieden.

Ein wichtiger Anwendungsbereich des Personenkühlelements ist die Kühlung bei MS-Patienten. Im Bereich des Leistungssports kann durch die Kühlungswirkung der Wasserverdunstung der Wirkungsgrad der Sportperson erhöht werden, da für eine vorgegebene Abkühlung weniger Schwitzwasser produziert werden muss. Schliesslich ist das Personenkühlelement auch für Berufspersonen geeignet, die einer erheblichen Wärmeeinwirkung ausgesetzt sind.

Das oben beschriebene Kühlelement wurde zwar als "Personenkühlelement" bezeichnet, doch könnte dieses auch zur Kühlung am tierischen Körper eingesetzt werden.

### Bezugszeichenliste

- 2: Kühlzone
- 4: Innenschicht
- 6: Aussenschicht
- 7: Elastische Mittel
- 8: Verdunstungsbereich
- 10: Körperoberfläche
- 12: Seitlicher Abschluss
- 14: Wasserleitung
- 16: Öffnung

## Patentansprüche

1. Personenkühlelement, insbesondere für Patienten, mit mindestens einer Kühlzone (2), wobei elastische Mittel (7) vorhanden sind, um die Kühlzone (2) gegen die Körperoberfläche (10) einer das Personenkühlelement tragenden Person vorzuspannen, wobei die Kühlzone (2) dreilagig ausgebildet ist mit einer körperzugewandten Innenschicht (4), einer körperabgewandten Aussenschicht (6) sowie einem dazwischen angeordneten Verdunstungsbereich (8), wobei die Innenschicht (4) und die Aussenschicht (6) je aus einem wasserdichten und wasserdampfdurchlässigen Material gebildet sind, und Wasserzuführmittel (14, 16) vorhanden sind, um den Verdunstungsbereich (8) mit flüssigem Wasser zu versorgen, **dadurch gekennzeichnet, dass** die Innenschicht (4) für Hautkontakt vorgesehen ist und dass der Verdunstungsbereich (8) aus einem hydrophilen Material gebildet ist.

2. Personenkühlelement nach Anspruch 1, **dadurch gekennzeichnet, dass** der Verdunstungsbereich (8) ein Kanalsystem beinhaltet.

3. Personenkühlelement nach einem der Ansprüche 1 oder 2, **dadurch gekennzeichnet, dass** die Aussenschicht (6) eine Dicke von 1 bis 5 µm aufweist.

4. Personenkühlelement nach einem der Ansprüche 1 bis 3, **dadurch gekennzeichnet, dass** sich die Innenschicht (4) eine Dicke von 10 bis 20 µm aufweist.

5. Personenkühlelement nach einem der Ansprüche 1 bis 4, **dadurch gekennzeichnet, dass** Innenschicht (4) und die Aussenschicht (6) zu einem seitlichen Abschluss (12) des Verdunstungsbereichs (8) verbunden sind.

6. Personenkühlelement nach einem der Ansprüche 1 bis 5, **dadurch gekennzeichnet, dass** es als Kleidungsstück ausgestaltet ist.

7. Personenkühlelement nach einem der Ansprüche 1 bis 6, **dadurch gekennzeichnet, dass** die Kühlzone (2) zum Anliegen an mindestens einer ausgewählten Körperpartie ausgebildet ist.

8. Personenkühlelement nach einem der Ansprüche 1 bis 7, **dadurch gekennzeichnet, dass** die Wasserzuführmittel ein mit einer Pumpe verbundenes Wasserleitungssystem (14) umfassen.

## Claims

1. Personal cooling element, particularly for patients, with at least one cooling zone (2), wherein elastic means (7) are provided to pretension the cooling zone (2) against the body surface (10) of a person wearing the personal cooling element, wherein the cooling zone (2) is three-layered with an internal layer (4) facing towards the body, an external layer (6) facing away from the body and an evaporation zone (8) arranged therebetween, wherein the internal layer (4) and the external layer (6) each are made from a material that is waterproof and permeable to water vapor, and wherein water supply means (14, 16) are present to supply the evaporation zone (8) with liquid water, **characterized in that** the evaporation zone (8) is made from a hydrophilic material.

2. Personal cooling element according to claim 1, **characterized in that** the evaporation zone (8) comprises a channel system.

3. Personal cooling element according to claim 1 or 2, **characterized in that** the external layer (6) has a thickness of 1 to 5 µm.

4. Personal cooling element according to any one of claims 1 to 3, **characterized in that** the internal layer (4) has a thickness of 10 to 20 µm.

5. Personal cooling element according to any one of claims 1 to 4, **characterized in that** the internal layer (4) and the external layer (6) are connected to form a lateral seal (12) of the evaporation zone (8).

6. Personal cooling element according to any one of claims 1 to 5, **characterized in that** it is formed as a garment.

7. Personal cooling element according to any one of claims 1 to 6, **characterized in that** the cooling zone (2) is formed for a tight fit to at least one selected body part.

8. Personal cooling element according to any one of claims 1 to 7, **characterized in that** the water supply means comprise a water supply system (14) connected to a pump.

## Revendications

1. Elément de refroidissement de personnes, en particulier de patients comportant au moins une zone de refroidissement (2), des moyens élastiques (7) permettant de serrer préalablement la zone de refroidissement (2) contre la surface (10) du corps d'une personne portant l'élément de refroidissement, la zone de refroidissement (2) étant réalisée en trois couches, à savoir une couche interne (4) tournée vers le corps, une couche externe (6) située à l'opposé du corps et une zone d'évaporation (8) située entre ces deux couches, la couche interne (4) et la couche externe (6) étant réalisées chacune en un matériau imperméable à l'eau et à la vapeur d'eau, et des moyens d'alimentation en eau (14, 16) permettant d'alimenter la zone d'évaporation (8) en eau liquide,
**caractérisé en ce que**
la couche interne (4) est prévue pour être en contact avec la peau et la zone d'évaporation (8) est réalisée en un matériau hydrophile.

2. Elément de refroidissement de personnes selon la revendication 1, **caractérisé en ce que**
la zone d'évaporation (8) renferme un système de canaux.

3. Elément de refroidissement de personnes selon l'une des revendications 1 et 2,
**caractérisé en ce que**
la couche externe (6) présente une épaisseur de 1 à 5 µm.

4. Elément de refroidissement de personnes selon l'une des revendications 1 à 3,
**caractérisé en ce que**
la couche interne (4) présente une épaisseur de 10 à 20 µm.

5. Elément de refroidissement de personnes selon l'une des revendications 1 à 4,
**caractérisé en ce que**
la couche interne (4) et la couche externe (6), sont reliées pour former un élément de fermeture latérale (12) de la zone d'évaporation (8).

6. Elément de refroidissement de personnes selon l'une des revendications 1 à 5,
**caractérisé en ce qu'**
il est réalisé sous la forme d'un élément de vêtement.

7. Elément de refroidissement de personnes selon l'une des revendications 1 à 6,
**caractérisé en ce que**
la zone de refroidissement (2) est réalisée pour être appliquée sur au moins une partie du corps sélectionnée.

8. Elément de refroidissement de personnes selon l'une des revendications 1 à 7,
**caractérisé en ce que**
les moyens d'alimentation en eau comportent un système de conduite d'eau (14) relié à une pompe.
